# EUROPEAN PATENT SPECIFICATION

(11) **EP 2 892 464 B1**
(45) Date of publication and mention of the grant of the patent: **09.11.2016**
(21) Application number: 13783397.6
(22) Date of filing: 03.09.2013
(51) Int. Cl.: A61F 2/14, G01N 21/95, G02B 21/00, G01N 21/51, A61B 3/107

(54) **APPARATUS AND METHOD FOR DETERMINING AN IMPLANT ORIENTATION OF A DONOR CORNEA LENS IN A RECEIVING STROMAL BED**
VORRICHTUNG UND VERFAHREN ZUR BESTIMMUNG EINER IMPLANTATORIENTIERUNG EINER HORNHAUT-DONATORLINSE IN EINEM EMPFANGENDEN STROMABETT
APPAREIL ET PROCÉDÉ DE DÉTERMINATION D'UNE ORIENTATION D'IMPLANT D'UNE LENTILLE CORNÉENNE DE DONNEUR DANS UN LIT STROMAL RECEVEUR

(30) Priority: 03.09.2012 IT MI20121468
(43) Date of publication of application: 15.07.2015
(73) Proprietor: Phronema S.r.l., Bari (IT)
(72) Inventor: LIPARI, Eugenio, Bari (IT); ALESSIO, Giovanni, Bari (IT)
(74) Representative: Bernotti, Andrea
(86) International application number: PCT/IB2013/058263
(87) International publication number: WO 2014/033697

(56) References cited:
- US-A- 5 920 373
- US-B1- 6 286 959
- US-B1- 6 594 021

## Description

### TECHNICAL FIELD

The present invention concerns an apparatus and a method for determining an implant orientation of a donor cornea lens in a receiving stromal bed.

### BACKGROUND ART

In ophthalmic surgery, cornea transplants are performed using two different techniques.

Perforating (or full thickness) transplants involve removal of the entire thickness of corneal tissue of the patient and complete replacement with a donor cornea. By an appropriate device, a circular portion of the central cornea of the receiving patient is removed with complete perforation. The tissue removed is integrally replaced with a lenticle of equivalent geometry, obtained from the donor cornea. The operation concludes with suturing (using various possible techniques) along the perimeter of the grafted tissue. After perforating transplant, the patient uses only the portion of cornea coming from the donor for vision.

In the case of lamellar transplants, on the other hand, only a certain thickness of corneal tissue is removed from the epithelial layer and from the endothelial layer of the receiving patient, using one of the various surgical techniques available. Lamellar transplants are classified, according to the residual thickness of the receiving tissue, into DALK (Deep Anterior Lamellar Keratoplasty) if the removal is deep and the residual tissue thickness of the receiver is modest, and ALK (Anterior Lamellar Keratoplasty) if the removal is more superficial and the residual tissue is thicker.

The removal of material allows the creation of a seat, normally called stromal bed or more simply receiving bed, in the patient's stromal tissue. The receiving stromal bed is shaped to receive a lenticle or stromal lens coming from a donor.

Irrespective of the surgical technique adopted to create the receiving bed, grafting of the donor stromal lens creates an interface between the receiving tissue and the donor tissue. The interface affects the properties of the resulting optical system.

In a significant number of cases, a lamellar cornea transplant, even when correctly performed, does not give the patient the expected refractive results in terms of improvement in the quality of vision. In particular, evaluation of the transplant performed by the usual instruments for objective ophthalmic examination is often not matched by the subjective perception of the patient, who does not experience the predicted improvements. Postoperative examinations show that, at times, the quality of vision is not satisfactory even when both the receiving bed and the donor lens are perfectly transparent according to the traditional ophthalmological examination methods. It has been furthermore ascertained that the visual result is all the more predictable the greater the quantity of tissue removed from the cornea of the receiver.

In the case of lamellar transplants, the properties of the final optical system are therefore determined not only by the quality of the residual receiving tissue and the donor tissue, but also by the way in which the donor lens is coupled with the receiving bed.

Document US 6 594,021 B describes an examination of a donor cornea in which polarised light is projected on the cornea (column 4, lines 27-30). During the examination, the cornea is contained in a vessel which is rotatable by a motor (column 3, lines 60-63, column 5, lines 40-45) in order to detect defects of the cornea, such as aberrations (column 4, lines 30-34).

### DISCLOSURE OF INVENTION

It is an object of the present invention is to provide an apparatus and a method for determining an implant orientation of a donor cornea lens in a receiving stromal bed, which allows the limitations described to be overcome and, in particular, allows improvement of the lamellar cornea transplant procedures and the quality of vision resulting from lamellar cornea transplant.

According to the present invention an apparatus and a method are provided for determining an implant orientation of a donor cornea lens in a receiving stromal bed, as defined in claims 1 and 13 respectively.

### BRIEF DESCRIPTION OF THE DRAWINGS

For a better understanding of the invention, some embodiments thereof will now be described, purely by way of non-limiting example and with reference to the accompanying drawings, in which:
- figure 1 shows a simplified block diagram of an apparatus for determining an implant orientation of a donor cornea lens in a receiving stromal bed in accordance with an embodiment of the present invention; and
- figure 2 shows a simplified block diagram of an apparatus for determining an implant orientation of a donor cornea lens in a receiving stromal bed in accordance with a different embodiment of the present invention.

### BEST MODE FOR CARRYING OUT THE INVENTION

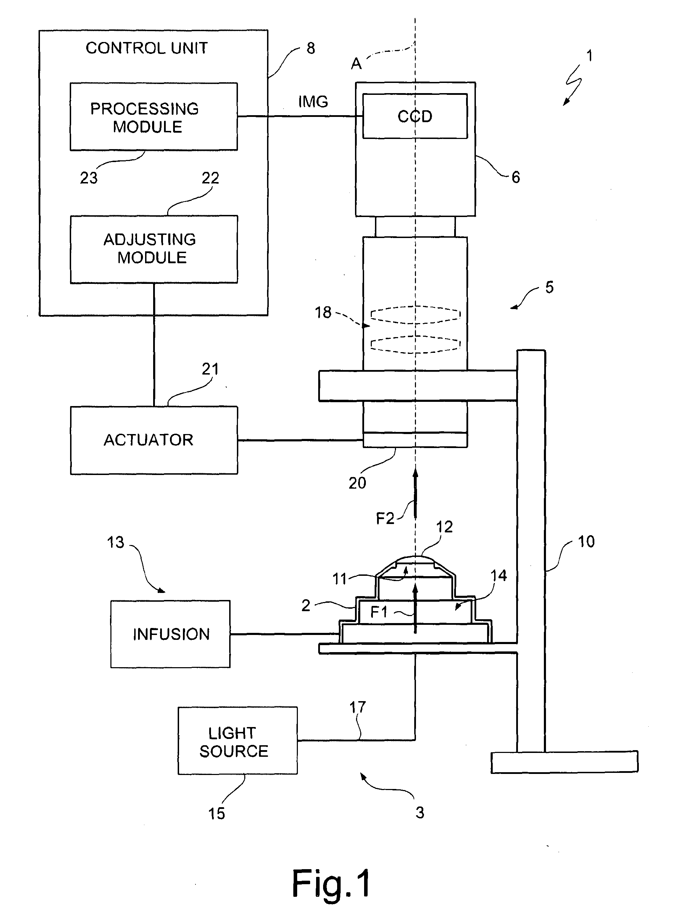

With reference to figure 1, an apparatus for determining an implant orientation of a donor cornea lens in a receiving stromal bed is indicated as a whole by the number 1.

The apparatus 1 comprises a supporting device 2, a lighting device 3, an optical unit 5, a light intensity detector 6 and a control unit 8.

The supporting device 2, the optical unit 5 and the light intensity detector device 6 are mounted on a frame 10 which ensures correct alignment along an optical axis A.

The supporting device 2 is provided with an observation seat 11 to receive a donor stromal lens 12 to be implanted in a receiving stromal bed (not shown). In particular, the observation seat 11 is configured so that the donor stromal lens 12 is supported in a position substantially perpendicular to the optical axis A.

In one embodiment, the supporting device 2 comprises an artificial front chamber 14, which allows the donor cornea lens 12 to be maintained in pressure conditions analogous to the post-implant pressure conditions. For this purpose, the supporting device 2 is provided with an infusion circuit 13 which feeds a saline solution at a controlled pressure into the artificial front chamber 14.

The lighting device 3 comprises a light source 15, coupled with the artificial front chamber 14 by an optic fibre guide 17. In this way, the inside of the artificial front chamber 14 is illuminated by a first light beam F1, with known intensity and wavelength, which is directed onto a rear face of the donor cornea lens 12 housed in the observation seat 11. The observation seat 11 is therefore arranged between the light source 15 and the light intensity detector device 6.

A second light beam F2 is transmitted through the donor cornea lens 12 along the optical axis A. In one embodiment, not illustrated, the lighting device directs the first light beam onto an anterior surface of the cornea and the supporting device is reflecting. The first light beam crosses the donor cornea lens and is reflected by the supporting device. The second light beam is transmitted through the donor cornea lens again along the optical axis, but in the opposite direction with respect to the first light beam.

The optical unit 5 comprises a focusing unit 18 and a polarizing filter 20.

The focusing unit 18, illustrated only schematically in figure 1, comprises a group of lenses (not shown in detail) and allows the second light beam F2 to be focused onto a detection surface of the light intensity detector device 6.

The polarizing filter 20 is arranged along the second light beam F2 and, in the embodiment described here, is perpendicular to the optical axis A. Furthermore, the polarizing filter 20 can be adjusted around its own optical axis which, in this case, coincides with the optical axis A. A polarization direction of the second light beam F2 coming out of the polarizing filter may therefore be selected by adjusting the orientation of the polarizing filter 20 itself with respect to the second ingoing light beam F2. It should be noted that, in a different embodiment, not illustrated, the polarizing filter may be arranged along the first light beam (i.e. before the interaction with the donor cornea lens).

An actuator 21, controlled by the control unit 8, allows adjustment of a relative angular position of the polarizing filter 20 with respect to the supporting device 2 and therefore with respect to the donor cornea lens 12 in the observation seat 11. In an alternative embodiment, the angular position of the polarizing filter 20 with respect to the supporting device 2 may be manually adjusted by an operator. In a further embodiment, adjustment members are incorporated in the supporting device and allow adjustment of the relative angular position of the supporting device itself, in particular of the observation seat which houses the donor cornea lens, and the polarizing filter.

The light intensity detector device 6 is coupled with the optical unit 5 so as to intercept the second light beam F2, which is focused on a detection surface. In one embodiment, the light intensity detector device 6 comprises a CCD image sensor and provides the control unit 8 with IMG image signals, representative of the donor cornea lens 12 in the observation seat 11, in the form of luminosity value matrixes. In a different embodiment, the light intensity detector device may comprise a photodetector or a group of photodetectors, such as phototransistors or photodiodes.

The control unit 8 is configured to acquire a plurality of light intensity values corresponding to respective orientations of the donor stromal lens 12 in relation to the polarizing filter 12 and to determine an implant orientation of the donor cornea lens 12 as a function of the acquired light intensity values.

In particular, the control unit 8 comprises an adjusting module 22 and a processing module 23.

The adjusting module 22 is configured to actuate the actuator 21 so as to arrange the polarizing filter 20 and the supporting device 2 (and therefore the donor cornea lens 12) in succession in a plurality of distinct relative angular positions.

The processing module 23 acquires a respective light intensity value for each relative angular position of the polarizing filter 20 with respect to the supporting device 2, processes the IMG image signals received from the light intensity detector device 6 in each relative angular position and determines, for each IMG image signal, a respective light intensity value, which is stored together with the corresponding relative angular position. In one embodiment, to determine the light intensity value, the processing module 23 isolates in each IMG image signal a portion corresponding to the observation seat 11, where the donor cornea lens 12 is placed, and calculates the average intensity thereof. The light intensity value thus determined is stored by the processing module 23 and associated to the relative angular position of the polarizing filter 20 with respect to the supporting device 2 and the donor cornea lens 12 arranged in the observation seat 11. Lastly the processing module 23 is configured to select a maximum value from the acquired light intensity values and the relative angular position corresponding to the maximum value.

The maximum value and its relative angular position define the implant orientation of the donor cornea lens 12.

The invention is based on observation of the structure of a human cornea.

The stroma occupies approximately 90% of the thickness of the human cornea and is mainly composed of collagen, which forms fibrils having diameter of approximately 25-30 nm. The fibrils are organised into wider bands or fibres called corneal lamellae. In each lamella, the collagen fibrils are immersed in a matrix rich in proteoglycans, glycoproteins, mineral salts and keratocytes and are arranged parallel to one another.

The corneal lamellae of the deeper layers of the stroma are not organised in an isotropic manner, but have a preferential orientation along the supero-inferior or nasal-temporal corneal meridians (therefore in one of two substantially perpendicular directions).

In the anterior portion of the cornea, on the other hand, the lamellae have a distribution such as to produce substantially isotropic effects in optical terms. Furthermore in the anterior stroma, numerous antero-posterior interconnections are present between the lamellae, and a certain percentage of lamellae that reach the limbus terminate at Bowman's membrane. Here, the collagen fibrils are arranged according to a substantially tangential configuration.

In the case of lamellar cornea transplants, the cornea lens taken from the donor will certainly contain a large part of deep tissue, organised in an anisotropic manner, but normally grafted with random orientation in the receiving bed. The optical interaction between the donor tissue and the receiving bed is therefore not predictable.

The lamellae belonging to the deep corneal layer, both of the donor and the receiver, produce diffraction effects. In an intact cornea the organisation of the lamellae of the deep stromal layers is coherent and the effects of the perpendicular lamellae compensate for one another. Optically, some lamellae produce diffractive phenomena when light passes through, whereas others behave like a polarizing filter able to annul the elements of disturbance, guaranteeing transparency of the system.

In the case of lamellar transplant, the transplanted lens, may cause phenomena of interference when optically interacting with the receiving bed. Although the receiving bed and the donor cornea lens may be considered optically without defects, coupling with non-optimal orientation may reduce the capacity of the resulting optical system to transmit the incident light.

The apparatus according to the invention allows identification of the orientation of the donor cornea lens (in particular, with respect to the polarizing filter) which guarantees conditions of maximum transparency. In particular, the orientation selected corresponds to the maximum light intensity value detected during variation of the relative angular position of the supporting device and the donor cornea lens arranged in the observation seat with respect to the polarizing filter. The implant may therefore be performed so as to respect the orientation of the corneal lamellae of the receiving bed, which is known from literature (i.e. along the supero-inferior and nasal-temporal meridians). The outcome of the operation is therefore more predictable and the visual perception of the patient corresponds to expectations.

The apparatus described allows to determine the implant orientation in an objective manner, without coupling with the receiving cornea.

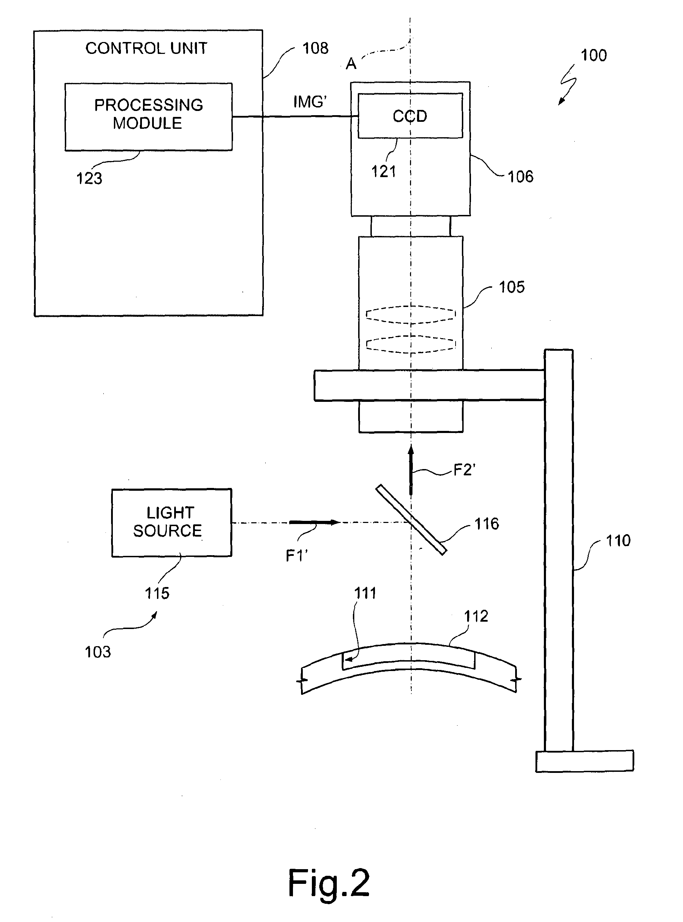

In a different embodiment of the invention, illustrated in figure 2, an apparatus 100 for determining the implant orientation of a donor cornea lens in a receiving stromal bed comprises a lighting device 103, an optical unit 105, a light intensity detector device 106 and a control unit 108.

The lighting device 103, the optical unit 105 and the light intensity detector device 106 are mounted on a frame 110. In particular, the frame 110 bears the lighting device 103 so that it is possible to illuminate a donor cornea lens 112 deposited (in a removable manner and without suture) in a receiving bed 111, which defines an observation seat. Analogously, the optical unit 105 and the light intensity detector device 106 are fixed to the frame 110 so that it is possible to set the donor cornea lens 112 in the receiving bed 111.

The lighting device 103 comprises a light source 115, which emits a first light beam F1' with known intensity and wavelength, and a semi-reflecting mirror 116, arranged so as to send the first light beam F1' towards the receiving bed 111 of the patient, where the donor cornea lens 112 is deposited.

The optical unit 105 is configured to focus a second light beam F2', coming from the donor cornea lens 112 arranged in the receiving bed 111, on a detection surface of the light intensity detector device 106.

The light intensity detector device 106 comprises an image sensor 121, for example a CCD sensor, which provides IMG' image signals representative of the donor cornea lens 112, in the form of light value matrixes. In particular, the IMG' image signals are formed of a second light beam F2', which is determined by the reflection of the first light beam F1' on the patient's eye structures (specifically iris and retina) and reaches the light intensity detector device 106 through the semi-reflecting mirror 116. The second light beam F2' is then transmitted through the cornea and its intensity is determined by the interaction both with the receiving bed 111 and with the donor cornea lens 112 arranged in the receiving bed 111. In particular, the intensity of the second light beam F2' is influenced by the angular position of the donor cornea lens 112 with respect to the receiving bed 111.

The control unit 108 is coupled with the image sensor 121 to receive the IMG' signals and comprises an image processing module 123 which is configured to acquire, in response to a user command, a plurality of images of the donor cornea lens 112 in the receiving bed 111, represented by respective IMG' image signals, and to determine the implant orientation of the donor cornea lens 112 from comparison between the acquired images. In the embodiment described, the angular position of the donor cornea lens 112 with respect to the receiving bed 111 is modified manually by an operator (for example a surgeon) between the acquisition of two successive images.

In one embodiment, the image processing module 123 identifies, in the IMG' image signal corresponding to each acquired image, a respective portion of image relative to the iris of the receiver and determines a respective light intensity value (for example a mean value). Furthermore, the image processing module 123 determines the implant orientation on the basis of the light intensity values of the acquired image portions (for example, by selecting the orientation to which the image with the maximum intensity value corresponds).

In this case, the apparatus 100 directly exploits the tissue of the receiving bed 111 and reduces the use of additional components (for example a polarizing filter).

The apparatus described with reference to figure 2 allows subjective evaluation of the implant orientation, since the donor lens is coupled with the receiving bed during the measurements.

In an alternative embodiment, however, the apparatus 100 may also comprise an adjustable polarizing filter, as already described with reference to figure 1. In this case, use of the polarizing filter allows determination of the implant orientation without repeatedly modifying the angular position of the donor cornea lens in the receiving bed.

Modifications and variations can be made to the apparatus and method described without departing from the scope of the present invention, as defined in the attached claims.

## Claims

1. Apparatus for determining an implant orientation of a donor cornea lens in a receiving stromal bed, comprising:
a lighting device (3; 103), configured to send a first light beam (F1; F1') to a donor cornea lens (12; 112) to be implanted in a stromal receiving bed of a patient, when the donor cornea lens (12; 112) is arranged in an observation seat (11; 111);
a light intensity detector device (6; 106), arranged so as to receive a second light beam (F2; F2'), transmitted through the donor cornea lens (12; 112) placed in the observation seat (11; 111) and illuminated by the first light beam (F2; F2');
a control unit (8; 108) coupled to the light intensity detector device (6; 106) and configured to acquire a plurality light intensity values, corresponding to respective orientations of the donor cornea lens (12; 112) ; the apparatus being **characterised in that** the control unit is configured to determine an implant orientation of the donor cornea lens (12; 112) as a function of the acquired light intensity values.

2. Apparatus according to claim 1, comprising a polarizing filter (20) along one of the first light beam (F1) and the second light beam (F2).

3. Apparatus according to claim 2, comprising supporting device (2), defining the observation seat (11; 111).

4. Apparatus according to claim 3, wherein the supporting device (2) comprises an artificial front chamber (14).

5. Apparatus according to claim 3 or 4, wherein the lighting device (3; 103) comprises a light source (15; 115) coupled to the supporting device (2) so that the observation seat (11; 111) is arranged between the light source (15; 115) and the light intensity detector device (6; 106).

6. Apparatus according to any one of claims 3 to 5, comprising adjusting elements (21) for adjusting a relative angular position of the polarizing filter (20) with respect to the supporting device (2).

7. Apparatus according to claim 6, wherein the adjusting elements (21) are controlled by the control unit (8; 108).

8. Apparatus according to claim 7, wherein the control unit (8; 108) comprises an adjusting module (22), configured to actuate the adjusting elements so as to place the polarizing filter (20) and the supporting device (2) in succession in a plurality of distinct relative angular positions.

9. Apparatus according to claim 8, wherein the control unit (8; 108) comprises a processing module (23) configured to acquire a respective light intensity value for each relative angular position, to store the acquired light intensity values and the corresponding relative angular positions and to select a maximum value from the acquired light intensity values and the corresponding relative angular position.

10. Apparatus according to claim 1 or 2, wherein the light intensity detector device (106) comprises an image sensor (121) and the control unit (108) comprises an image processing module (123) coupled to the image sensor (121).

11. Apparatus according to claim 10, wherein the image processing module (123) is configured to acquire a plurality of images of the donor cornea lens (12; 112) and to determine the implant orientation from comparison of the acquired images.

12. Apparatus according to claim 11, wherein the image processing module (123) is configured to identify, in each acquired image, a respective image portion relating to an iris, to determine a respective light intensity value for each image portion and to determine the implant orientation on the basis of the light intensity values of the image portions.

13. Method for determining an implant orientation of a donor cornea lens in a receiving stromal bed, comprising:
illuminating a donor cornea lens (12; 112), to be implanted in a receiving stromal bed of a patient, with a first light beam (F1; F1');
detecting an intensity of a second light beam (F2; F2'), transmitted through the donor cornea lens (12; 112) illuminated by the first light beam (F1; F1');
acquiring a plurality of light intensity values of the second light beam (F2; F2'), corresponding to respective orientations of the donor cornea lens (12; 112);
the method being **characterised by**
determining the implant orientation of the donor cornea lens (12; 112) as a function of the acquired light intensity values.

14. Method according to claim 13, comprising using a light source (15; 115), a light intensity detector device (6; 106) and a polarizing optical element (20; 111) arranged between the light source (15; 115) and the light intensity detector device (6; 106).

15. Method according to claim 14, comprising placing the polarizing filter (20) and the supporting device (2) in succession in a plurality of distinct relative angular positions and acquiring a respective light intensity value foe each relative angular position.

16. Method according to claim 15, wherein determining the implant orientation of the donor cornea lens (12; 112) as a function of the acquired light intensity values comprises selecting a maximum value from the acquired light intensity values and the corresponding relative angular position.

## Patentansprüche

1. Vorrichtung zur Bestimmung einer Implantat-Orientierung einer Spender-hornhautlinse in einem Empfänger-Stromabett bzw. aufnehmenden Stromabett, welche Folgendes aufweist:
eine Beleuchtungseinrichtung (3; 103), die konfiguriert ist, um einen ersten Lichtstrahl (F1, F1') auf eine Spenderhornhautlinse (12; 112) zu senden,
welche in einem aufnehmenden Stromabett eines Patienten implantiert werden soll, wenn die Spenderhornhautlinse (12; 112) in einem Beobachtungssitz (11; 111) angeordnet ist;
eine Lichtintensitätsdetektoreinrichtung (6; 106), die so angeordnet ist, dass sie einen zweiten Lichtstrahl (F2; F2') empfängt, der durch die Spenderhornhautlinse (12; 112) gesendet wird, welche in dem Beobachtungssitz (11; 111) angeordnet und von dem ersten Lichtstrahl (F1; F1') beleuchtet wird;
eine Steuereinheit (8; 108), die mit der Lichtintensitätsdetektoreinrichtung (6; 106) gekoppelt ist und konfiguriert ist, um eine Vielzahl von Lichtintensitätswerten zu erfassen, und zwar entsprechend den jeweiligen Orientierungen der Spenderhornhautlinse (12; 112),
wobei die Vorrichtung **dadurch gekennzeichnet ist, dass** die Steuereinheit konfiguriert ist, um eine Implantat-Orientierung der Spenderhornhautlinse (12; 112) als eine Funktion der erfassten Lichtintensitätswerte zu bestimmen.

2. Vorrichtung nach Anspruch 1, welche einen Polarisationsfilter (20) entweder entlang dem ersten Lichtstrahl (F1) oder dem zweiten Lichtstrahl (F2) aufweist.

3. Vorrichtung nach Anspruch 2, die eine Trageinrichtung (2) aufweist, die den Beobachtungssitz (11; 111) definiert.

4. Vorrichtung nach Anspruch 3, wobei die Trageinrichtung (2) eine künstliche vordere Kammer (14) aufweist.

5. Vorrichtung nach Anspruch 3 oder 4, wobei die Beleuchtungseinrichtung (3; 103) eine Lichtquelle (15; 115) aufweist, die mit der Trageinrichtung (2) so gekoppelt ist, dass der Beobachtungssitz (11; 111) zwischen der Lichtquelle (15; 115) und der Lichtintensitätsdetektoreinrichtung (6; 106) angeordnet ist.

6. Vorrichtung nach einem der Ansprüche 3 bis 5, welche Einstellelemente (21) aufweist, um eine relative Winkelposition des Polarisationsfilters (20) in Bezug auf die Trageinrichtung (2) einzustellen.

7. Vorrichtung nach Anspruch 6, wobei die Einstellelemente (21) von der Steuereinheit (8; 108) gesteuert werden.

8. Vorrichtung nach Anspruch 7, wobei die Steuereinheit (8; 108) ein Einstellmodul (22) aufweist, das konfiguriert ist, um die Einstellelemente zu betätigen, um den Polarisationsfilter (20) und die Trageinrichtung (2) nacheinander in eine Vielzahl von unterschiedlichen relativen Winkelstellungen zu platzieren.

9. Vorrichtung nach Anspruch 8, wobei die Steuereinheit (8; 108) ein Verarbeitungsmodul (23) aufweist, das konfiguriert ist, um einen entsprechenden Lichtintensitätswert für jede relative Winkelposition zu erfassen, um die erfassten Lichtintensitätswerte und die entsprechenden relativen Winkelpositionen zu speichern und um einen Maximalwert aus den erfassten Lichtintensitätswerten und den entsprechenden relativen Winkelpositionen zu wählen.

10. Vorrichtung nach Anspruch 1 oder 2, wobei die Lichtintensitätsdetektoreinrichtung (106) einen Bildsensor (121) aufweist und die Steuereinheit (108) ein Bildverarbeitungsmodul (123) aufweist, das mit dem Bildsensor (121) gekoppelt ist.

11. Vorrichtung nach Anspruch 10, wobei das Bildverarbeitungsmodul (123) konfiguriert ist, um eine Vielzahl von Bildern der Spenderhornhautlinse (12; 112) zu erfassen und um die Implantat-Orientierung aus einem Vergleich der erfassten Bilder zu bestimmen.

12. Vorrichtung nach Anspruch 11, wobei das Bildverarbeitungsmodul (123) konfiguriert ist, um in jedem erfassten Bild, einen jeweiligen Bildausschnitt bezüglich einer Iris zu identifizieren, um einen entsprechenden Lichtintensitätswert für jeden Bildausschnitt zu bestimmen und um die Implantat-Orientierung auf der Grundlage der Lichtintensitätswerte der Bildausschnitte zu bestimmen.

13. Verfahren zum Bestimmen einer Implantat-Orientierung einer Spenderhornhautlinse in einem aufnehmenden Stromabett, welches Folgendes aufweist:
Beleuchten einer Spenderhornhautlinse (12; 112), die in einem aufnehmenden Stromabett eines Patienten implantiert werden soll, mit einem ersten Lichtstrahl (F1; F1');
Detektieren einer Intensität eines zweiten Lichtstrahls (F2; F2'), der durch die Spenderhornhautlinse (12; 112) gesendet wird, welche von dem ersten Lichtstrahl (F1; F1') beleuchtet wird;
Erfassen einer Vielzahl von Lichtintensitätswerten des zweiten Lichtstrahls (F2; F2'), die den jeweiligen Orientierungen der Spenderhornhautlinse (12; 112) entspricht;
wobei das Verfahren **gekennzeichnet ist durch**
Bestimmen der Implantat-Orientierung der Spenderhornhautlinse (12; 112) als eine Funktion der erfassten Lichtintensitätswerte.

14. Verfahren nach Anspruch 13, welches Folgendes aufweist: Verwenden einer Lichtquelle (15; 115), einer Lichtintensitätsdetektoreinrichtung (6; 106) und eines polarisierenden optischen Elementes (20; 111), das zwischen der Lichtquelle (15; 115) und der Lichtintensitätsdetektoreinrichtung (6; 106) angeordnet ist.

15. Verfahren nach Anspruch 14, welches aufweist, den Polarisationsfilter (20) und die Trageinrichtung (2) nacheinander in eine Vielzahl von unterschiedlichen relativen Winkelpositionen zu platzieren und einen entsprechenden Lichtintensitätswert für jede relative Winkelposition zu erfassen.

16. Verfahren nach Anspruch 15, wobei das Bestimmen der Implantat-Orientierung der Spenderhornhautlinse (12; 112) als eine Funktion der erfassten Lichtintensitätswerte aufweist, einen Maximalwert aus den erfassten Lichtintensitätswerten und der entsprechenden relativen Winkelposition auszuwählen.

## Revendications

1. Appareil destiné à déterminer l'orientation d'un implant de lentille cornéenne de donneur dans un lit stromal receveur, comprenant :
un dispositif d'éclairage (3 ; 103) conçu pour envoyer un premier faisceau de lumière (F1 ; F1') à une lentille cornéenne de donneur (12 ; 112) à implanter dans un lit stromal receveur de patient, lorsque la lentille cornéenne de donneur (12 ; 112) est disposée sur un socle d'observation (11 ; 111) ;
un dispositif de détection d'intensité lumineuse (6 ; 106) disposé de manière à recevoir un second faisceau de lumière (F2 ; F2') transmis à travers la lentille cornéenne de donneur (12 ; 112) placée sur le socle d'observation (11 ; 111) et éclairée par le premier faisceau de lumière (F1 ; F1') ;
une unité de commande (8 ; 108) couplée au dispositif de détection d'intensité lumineuse (6 ; 106) et configurée pour acquérir une pluralité de valeurs d'intensité lumineuse correspondant à des orientations respectives de la lentille cornéenne de donneur (12 ; 112),
l'appareil étant **caractérisé en ce que** l'unité de commande est configurée pour déterminer une orientation d'implant de la lentille cornéenne de donneur (12 ; 112) en fonction des valeurs d'intensité lumineuse acquises.

2. Appareil selon la revendication 1, comprenant un filtre de polarisation (20) le long de l'un du premier faisceau de lumière (F1) et du second faisceau de lumière (F2).

3. Appareil selon la revendication 2, comprenant un dispositif de support (2) définissant le socle d'observation (11 ; 111).

4. Appareil selon la revendication 3, dans lequel le dispositif de support (2) comprend une chambre antérieure artificielle (14).

5. Appareil selon la revendication 3 ou 4, dans lequel le dispositif d'éclairage (3 ; 103) comprend une source lumineuse (15 ; 115) couplée au dispositif de support (2) de manière que le socle d'observation (11 ; 111) soit disposé entre la source lumineuse (15 ; 115) et le dispositif de détection d'intensité lumineuse (6 ; 106).

6. Appareil selon l'une quelconque des revendications 3 à 5, comprenant des éléments de réglage (21) destinés à régler une position angulaire relative du filtre de polarisation (20) par rapport au dispositif de support (2).

7. Appareil selon la revendication 6, dans lequel les éléments de réglage (21) sont commandés par l'unité de commande (8 ; 108).

8. Appareil selon la revendication 7, dans lequel l'unité de commande (8 ; 108) comprend un module de réglage (22) configuré pour actionner les éléments de réglage de manière à placer le filtre de polarisation (20) et le dispositif de support (2) tour à tour à une pluralité de positions angulaires relatives distinctes.

9. Appareil selon la revendication 8, dans lequel l'unité de commande (8 ; 108) comprend un module de traitement (23) configuré pour acquérir une valeur d'intensité lumineuse respective pour chaque position angulaire relative, pour stocker les valeurs d'intensité lumineuse acquises et les positions angulaires relatives correspondantes, et pour sélectionner une valeur maximale parmi les valeurs d'intensité lumineuse acquises et la position angulaire relative correspondante.

10. Appareil selon la revendication 1 ou 2, dans lequel le dispositif de détection d'intensité lumineuse (106) comprend un capteur d'images (121) et l'unité de commande (108) comprend un module de traitement d'images (123) couplé au capteur d'images (121).

11. Appareil selon la revendication 10, dans lequel le module de traitement d'images (123) est configuré pour acquérir une pluralité d'images de la lentille cornéenne de donneur (12 ; 112) et pour déterminer l'orientation de l'implant par comparaison des images acquises.

12. Appareil selon la revendication 11, dans lequel le module de traitement d'images (123) est configuré pour identifier, sur chaque image acquise, une partie d'image respective se rapportant à un iris, pour déterminer une valeur d'intensité lumineuse respective de chaque partie d'image et pour déterminer l'orientation de l'implant sur la base des valeurs d'intensité lumineuse des parties d'image.

13. Procédé pour déterminer une orientation d'implant de lentille cornéenne de donneur dans un lit stromal receveur, comprenant les étapes consistant à :
éclairer une lentille cornéenne de donneur (12 ; 112) à implanter dans un lit stromal receveur de patient, avec un premier faisceau de lumière (F1 ; F1') ;
détecter une intensité d'un second faisceau de lumière (F2 ; F2') transmis à travers la lentille cornéenne de donneur (12 ; 112) éclairée par le premier faisceau de lumière (F1 ; F1') ;
acquérir une pluralité de valeurs d'intensité lumineuse du second faisceau de lumière (F2 ; F2') correspondant à des orientations respectives de la lentille cornéenne de donneur (12 ; 112) ;
le procédé étant **caractérisé par** :
la détermination de l'orientation de l'implant de lentille cornéenne de donneur (12 ; 112) en fonction des valeurs d'intensité lumineuse acquises.

14. Procédé selon la revendication 13, comprenant l'utilisation d'une source lumineuse (15 ; 115), d'un dispositif de détection d'intensité lumineuse (6 ; 106) et d'un élément optique de polarisation (20 ; 111) disposé entre la source lumineuse (15 ; 115) et le dispositif de détection d'intensité lumineuse (6 ; 106).

15. Procédé selon la revendication 14, comprenant le positionnement du filtre de polarisation (20) et du dispositif de support (2) tour à tour à une pluralité de positions angulaires relatives distinctes et l'acquisition d'une valeur d'intensité lumineuse respective pour chaque position angulaire relative.

16. Procédé selon la revendication 15, dans lequel la détermination de l'orientation de l'implant de lentille cornéenne de donneur (12 ; 112) en fonction des valeurs d'intensité lumineuse acquises comprend la sélection d'une valeur maximale parmi les valeurs d'intensité lumineuse acquises et de la position angulaire relative correspondante.
